# EUROPEAN PATENT SPECIFICATION

(11) **EP 1 755 581 B1**
(45) Date of publication and mention of the grant of the patent: **15.07.2015**
(21) Application number: 05735674.3
(22) Date of filing: 26.04.2005
(51) Int. Cl.: A61K 31/195, A61K 31/137, A61K 31/138, A61P 9/04

(54) **METHODS AND COMPOSITIONS FOR THE TREATMENT OF MYOCARDIAL CONDITIONS**
VERFAHREN UND ZUSAMMENSETZUNGEN ZUR BEHANDLUNG VON MYOKARDIALEN ERKRANKUNGEN
METHODES ET COMPOSITIONS DE TRAITEMENT D'ETATS MYOCARDIQUES

(30) Priority: 23.04.2004 AU 2004902179
(43) Date of publication of application: 28.02.2007
(73) Proprietor: Northern Sydney Local Health District, St. Leonards, NSW 2065 (AU)
(72) Inventor: RASMUSSEN, Helge, H., Mosman, NSW 2088 (AU); BUNDGAARD, Henning, DK-2840 Holte (DK)
(74) Representative: Ryan, Anne Mary
(86) International application number: PCT/AU2005/000590
(87) International publication number: WO 2005/102304

(56) References cited:
- EP-A2- 1 145 717
- WO-A-00/18389
- WO-A-02/087508
- WO-A-02/094820
- WO-A2-01/07025
- US-A1- 2004 053 852
- US-B1- 6 344 474
- HORINOUCHI TAKAHIRO ET AL: "Structure-activity relationship studies of (+-)-terbutaline and (+-)-fenoterol on beta3-adrenoceptors in the guinea pig gastric fundus" JOURNAL OF SMOOTH MUSCLE RESEARCH, vol. 37, no. 3-4, August 2001 (2001-08), pages 105-112, XP002458606 ISSN: 0916-8737
- DE GROOT ANNEMIEKE A ET AL: "Involvement of the beta3 adrenoceptor in nebivolol-induced vasorelaxation in the rat aorta." JOURNAL OF CARDIOVASCULAR PHARMACOLOGY, vol. 42, no. 2, August 2003 (2003-08), pages 232-236, XP009092210 ISSN: 0160-2446
- CARSON P E: "Beta-blocker therapy in heart failure: pathophysiology and clinical results." CURRENT PROBLEMS IN CARDIOLOGY JUL 1999, vol. 24, no. 7, July 1999 (1999-07), pages 421-460, XP002458608 ISSN: 0146-2806
- KOZLOVSKI V I ET AL: "EFFECTS OF TWO BETA3-AGONISTS, CGP 12177A AND BRL 37344, ON CORONARY FLOW AND CONTRACTILITY IN ISOLATED GUINEA PIG HEART" JOURNAL OF CARDIOVASCULAR PHARMACOLOGY, NEW YORK, NY, US, vol. 41, no. 5, May 2003 (2003-05), pages 706-713, XP009056377 ISSN: 0160-2446
- MONIOTTE S ET AL: "The [beta]3-adrenoceptor and its regulation in cardiac tissue" INTENSIVMEDIZIN UND NOTFALLMEDIZIN 2003 GERMANY, vol. 40, no. 6, 2003, pages 484-493, XP002458609 ISSN: 0175-3851
- LOHSE M J ET AL: "What Is the Role of [beta]-Adrenergic Signaling in Heart Failure?" CIRCULATION RESEARCH 14 NOV 2003 UNITED STATES, vol. 93, no. 10, 14 November 2003 (2003-11-14), pages 896-906, XP002458610 ISSN: 0009-7330
- CHENG HENG-JIE ET AL: "Functional significance of beta3-adrenergic receptor activation in heart failure" JOURNAL OF MOLECULAR AND CELLULAR CARDIOLOGY, vol. 33, no. 6, June 2001 (2001-06), page A20, XP002458611 & XVII ISHR WORLD CONGRESS OF THE INTERNATIONAL SOCIETY FOR HEART RESEARCH; WINNIPEG, CANADA; JULY 06-11, 2001 ISSN: 0022-2828
- KARLE A. CHRISTOPHER ET AL: 'Antiarrhytmic drug carvedilol inhibits HERG patassium channels.' CARDIOVASCULAR RESEARCH. vol. 49, no. 2, 2001, pages 361 - 370, XP008090670
- WHEELDON N.M. ET AL: 'Cardiac effects of the beta 3-adrenoceptor agonist BRL35135 in man.' BR J CLIN PHARMAC. vol. 37, no. 4, 1994, pages 363 - 369, XP009092055
- CARSON PETER E. ET AL: 'Beta-blocker therapy in heart failure: pathophysiology and clinical results.' CURRENT PROBLEMS IN CARDIOLOGY. vol. 24, no. 7, July 1999, pages 421 - 460, XP002458608
- 'Structures of some compounds mentioned in the application' 02 July 2014, XP055148438

## Description

### Technical Field

The present invention relates to methods for the treatment of conditions characterised by abnormalities of myocardial cell ion levels, such as Na⁺, K⁺ and Ca²⁺ ions. The present invention also relates to methods of treatment of heart failure and myocardial hypertrophy. The invention also relates to compounds and compositions useful in the treatment of mammals, particularly humans, having a condition characterised by abnormalities of myocardial cell ion levels, such as Na⁺, K⁺ and Ca²⁺ ions, including heart failure and myocardial hypertrophy.

### Background

There are a number of conditions affecting humans and other animals which are characterised by abnormalities of myocardial cell ion levels, such as Na⁺, K⁺ and Ca²⁺ ions. Such conditions include heart failure, myocardial hypertrophy and hypertrophic cardiomyopathy.

In the Western world the prevalence of symptomatic heart failure is approximately 2%. In the United States ~5 million people are treated for it and it is estimated that 50-60 million Americans (∼20%) are at risk of developing heart failure (Gheorghiade *et al.,* 2003). This group includes people suffering from one or more of the leading causes for development of heart failure, *i.e.* coronary artery disease, diabetes, hypertension or valvular heart disease.

Heart failure (HF), or congestive heart failure, represents a complex clinical syndrome characterised by abnormalities of left ventricular function and neurohormonal regulation. In general terms it is a condition characterised by reduced pumping capacity of the heart, which compromises perfusion of the body, initially typically during exertion, and with worsening HF also at rest. The reduced pumping capacity can be due to reduced contractile force of the heart muscle, the myocardium (systolic failure), or due to compromised re-filling of the heart, *i.e.* compromised myocardial relaxation (diastolic failure). HF develops due to a wide spectrum of aetiological and pathophysiological factors and varies considerably in clinical presentation.

The symptoms generally seen in HF are exertional dyspnoea, chest discomfort, orthopnoea, reduced exercise capacity, fluid retention causing weight gain, anorexia and nausea, fatigue and weakness, and in advanced stages central nervous system symptoms, cachexia and reduced longevity. However, even severely reduced pump function may be associated with none or only few symptoms.

The neurohormonal abnormalities characteristic of HF include raised serum- and tissue levels of angiotensin II, catecholamines, in particular norepinephrine (noradrenaline), aldosterone, endothelin and natriuretic peptides. Addressing these abnormalities has been the foundation for clinical trials of pharmacological treatment. Drugs that antagonise the effect of angiotensin II (receptor blockers) or prevent its synthesis, angiotensin converting enzyme (ACE) inhibitors, aldosterone antagonists and β-adrenoceptor blockers significantly reduce morbidity and mortality in HF. Trials of endothelin receptor blockers and of drugs designed to raise levels of natriuretic peptides have not shown benefit.

In myocardial hypertrophy the increase in ventricular mass is paralleled by myocyte hypertrophy, i.e. increased size of the heart cells. Clinically, myocardial hypertrophy is often diagnosed by electrocardiography or by echocardiography showing an increased ventricular wall thickness. However, if the heart is also dilated, hypertrophy may be present with normal or even thin ventricular walls. Hypertrophy most often develops as a compensatory mechanism that helps the heart to deal with an increase in workload, e.g. due to heart valve disease or arterial hypertension. Myocardial hypertrophy may also develop due to abnormalities in the genes encoding the contractile proteins in the myocyte (Bundgaard *et al*., 1999). This disease entity is associated with considerable morbidity and a high mortality rate.

In a recent meta-analysis of the prognostic implications of myocardial hypertrophy, Vakili *et al.* (2001) reported that myocardial hypertrophy consistently predicts a high risk for morbidity as well as mortality, independently of examined covariates, with no clear difference in relation to race, presence or absence of hypertension or coronary disease, or between clinical and epidemiologic samples. These results clarify the strong relation between ventricular hypertrophy and adverse outcome and emphasize the clinical importance of its detection. A meta-analysis of the outcome of medical management of this condition concluded that regression of left ventricular hypertrophy during antihypertensive treatment is associated with a marked reduction in risk for subsequent cardiovascular disease (Verdecchia *et al,* 2003).

WO 02/094820 relates to crystalline salts of 2-(4-{2-[2-hydroxy-3-(2-thiophen-2-yl-phenoxy)-propylamino]-2-methyl-propyl}-phenoxy)-nicotinonitrile. The salts of WO 02/094820 are described as beta 3 adrenergic receptor agonists which are capable of increasing lipolysis and energy expenditure in cells and, therefore, are useful, e.g., for treating type 2 diabetes and/or obesity.

According to US 2004/053852, desensitization of receptors that control disease is prevented by inhibiting G-protein receptor kinases. This has applicability, e.g., for patients with heart failure or on a left ventricular heart device or a heart pump after surgery or about to undergo surgery and at high risk for a cardiac event or on an opiate or addicted to opiate or with cystic fibrosis or rheumatoid arthritis.

WO 00/18389 describes a method of using a beta 2 adrenergic receptor agonist that selectively activates GS proteins in the treatment of cardiovascular disease. More particularly, WO 00/18389 describes a method of using fenoterol or an acid addition salt thereof to activate beta 2AR GS proteins selectively in the treatment of acute heart failure, chronic heart failure, aging heart and the like.

WO 02/087508 describes nitrosated and/or nitrosylated nebivolol, nitrosated and/or nitrosylated metabolites of nebivolol and compositions comprising at least one nitrosated and/or nitrosylated nebivolol and/or at least one nitrosated and/or nitrosylated metabolite of nebivolol, and optionally, at least one nitric oxide donor and/or at least one antioxidant or a pharmaceutically acceptable salt thereof, and/or at least one compound used to treat cardiovascular disease or a pharmaceutically acceptable salt thereof, and/or at least one nitrosated compound used to treat cardiovascular diseases.

### Excitation-contraction coupling in the heart

In response to the cardiac action potential Ca²⁺ ions enter the cell from the outside and "trigger" release of Ca²⁺ from the sarcoplasmic reticulum (SR). The Ca²⁺ interact with contractile proteins of the cell and initiate contraction of the heart.

Relaxation of the heart depends on removal of Ca²⁺. This occurs by re-uptake of Ca²⁺ into the SR and by extrusion across the cell membrane. Extrusion of Ca²⁺ across the cell membrane occurs mainly *via* an exchange mechanism that transports Ca²⁺ out in exchange for Na⁺ that is transported in.

Abnormalities in cellular handling of Ca²⁺ during excitation-contraction coupling are involved in the pathogenesis of HF and myocardial hypertrophy. Raised intracellular levels of Na⁺ in HF and myocardial hypertrophy is associated with a decrease in the electrochemical driving force that extrudes Ca²⁺ in exchange for Na⁺. The high Na⁺ levels may also be responsible for abnormalities in contraction.

The "uphill" transport of Ca²⁺ ions out of cells, against their electrochemical gradient, depends on the energy stored in an oppositely directed electrochemical gradient for Na⁺. The transmembrane Na⁺ gradient, in turn, is maintained by the Na⁺-K⁺ pump, effectively the only export route for Na⁺. The pump transports 3 Na⁺ ions out of cells in exchange for 2 K⁺ ions that are transported in.

The mortality in HF is extremely high and increases with age and co-morbidities. In a study of 38,000 patients with a first admission for HF one year mortality was 33.1% (Jong P *et al*, 2002). The overall 5-year mortality for all patients after the HF diagnosis is established is ∼50%. Despite a marked progress in the pharmacological management of HF the 5-year mortality has only come down from 60-70% in the period 1950-1969 to 50-60% in the period 1990-1999 (Levy D, 2002). Morbidity and mortality are thus still very high in heart failure despite administration of presently available drugs with proven efficacy.

Similar to the situation with heart failure, whilst there are treatments available for other conditions characterised by abnormalities of cellular ion levels each of the available treatments suffers one or more disadvantages and would benefit from the availability of improved methods of treatment.

Accordingly, there is a need for improved methods for the treatment of conditions characterised by abnormalities of myocardial cell ion levels, in particular abnormalities of cellular Na⁺, K⁺ or Ca²⁺ ions. In a particular focus, there is a need for improved methods for the treatment of HF and myocardial hypertrophy, and for improved agents and treatment regimes for HF and myocardial hypertrophy

### Summary of the Invention

The present invention aims to provide improved compositions for use in the treatment of conditions characterised by abnormalities of myocardial cell ion levels, in particular abnormalities of cellular Na⁺, K⁺ or Ca²⁺ ions and so to substantially alleviate deficiencies of current treatments for such conditions. In particular the present invention aims to provide improved compositions for use in the treatment of symptomatic heart failure and so to substantially alleviate deficiencies of current treatments.

The present invention is based on the surprising discovery by the inventors that a myocardial cell surface receptor, the β₃ adrenoceptor, activates the membrane Na⁺-K⁺ pump and is associated with extrusion of Na⁺ from myocardial cells.

According to the present invention there is provided a composition comprising a therapeutically effective amount of one or more β₃ adrenoceptor selective agonists having preferential stimulatory activity toward the β₃ andrenoreceptor for use in the treatment of a human suffering from symptomatic heart failure, the use further comprising use of one or more β blockers.

Also described herein is a method for the treatment of an individual suffering from symptomatic heart failure, said method comprising administering a therapeutically effective amount of one or more β₃ adrenoceptor selective agonists, having preferential stimulatory activity toward the β₃ andrenoreceptor, to said individual, the method further comprising administering one or more β blockers to said individual.

The individual may be an individual suffering from one or more clinical symptoms of symptomatic heart failure.

In a specific aspect of the invention the β₃ adrenoceptor selective agonist is selected from the β₃ adrenoceptor agonist groups arylethanolamines, aryloxypropanolamines, trimetoquinols.
In a specific aspect of the invention the β₃ adrenoceptor selective agonist may be selected from the group consisting of BRL37344, , N-5984, (R)-N-[4-[2-[[2-Hydroxy-2-(pyridin-3-yl)ethyl]amino]ethyl]phenyl]-4-[4-(4-trifluoro-methylphenyl)thiazol-2-yl]benzenesulfonamide (L-796568), (R)-N-[4-[2-[[2-hydroxy-2-(3-pyridinyl)-ethyl]amino]ethyl]phenyl]-1-(4-octylthiazol-2-yl)-5-indolinesulfonamide (L-755507), L-770,644, L-766,892, L-757,793, LY-377604, SR 58611A, and beta 3 agonist series 2-(3-indolyl) alkylamino-1-1(3-chlorophenyl)ethanols.

In a specific aspect of the invention the β₃ adrenoceptor selective agonist further comprises β₁ antagonist activity.

In a specific aspect of the invention the β₃ adrenoceptor selective agonist further comprises β₂ antagonist activity.

In a specific aspect of the invention the β blocker is a β₁ adrenoceptor antagonist.

In a specific aspect of the invention the β blocker may be administered to said individual prior to, simultaneously with or subsequent to administration of the one or more β₃ adrenoceptor selective agonists.

The method may comprise at least partially stabilizing said individual prior to administration of said β₃ adrenoceptor selective agonist.

The method may comprise commencement of a β₃ adrenoceptor selective agonist as an initial pharmacological approach in the treatment.

Said stabilizing may comprise treatment with one or more compounds selected from the group consisting of ACE-inhibitors, aldosterone antagonists and β adrenoceptor antagonists.

Also described herein is use of one or more β₃ adrenoceptor selective agonists having preferential stimulatory activity toward the β₃ andrenoreceptor in the manufacture of a medicament for the treatment of an individual suffering from symptomatic heart failure, further comprising use of one or more β blockers.

Also described herein is one or more β₃ adrenoceptor selective agonists having preferential stimulatory activity toward the β₃ andrenoreceptor for use in the treatment of an individual suffering from symptomatic heart failure, the use further comprising use of one or more β blockers.

Further described herein is a pharmaceutical composition for use in the treatment of symptomatic heart failure, the composition comprising one or more β₃ adrenoceptor selective agonists having preferential stimulatory activity toward the β₃ andrenoreceptor together with one or more pharmaceutically acceptable adjuvants, excipients and/or carriers, the use further comprising use of one or more β blockers.

The pharmaceutical composition may comprise one or more β₃ adrenoceptor selective agonists and one or more β₁ and/or β₂ adrenoceptor antagonists, together with one or more pharmaceutically acceptable adjuvants, excipients and/or carriers.

Further described is a method for the extrusion of Na⁺ from a myocardial cell or cells, the method comprising contacting said cell(s) with one or more β₃ adrenoceptor selective agonist(s). The method may comprise Na,K pump stimulation. The method may be an *in vitro* or an *in vivo* method.

### Definitions

The term "therapeutically effective amount" as used herein includes within its meaning a non-toxic but sufficient amount of a compound or composition for use in the invention to provide the desired therapeutic effect. The exact amount required will vary from subject to subject depending on factors such as the species being treated, the age and general condition of the subject, co-morbidities, the severity of the condition being treated, the particular agent being administered and the mode of administration and so forth. Thus, it is not possible to specify an exact "effective amount". However, for any given case, an appropriate "effective amount" may be determined by one of ordinary skill in the art using only routine methods.

In the context of this specification, the general class of receptors known as "beta (β) adrenoceptors" is relevant. In this context "beta adrenoceptor" or "β adrenoceptor" is understood to have its standard meaning in the art which, generally stated, includes β₁ adrenoceptors, β₂ adrenoceptors, β₃ adrenoceptors, which are receptors for circulating catecholamines (adrenaline and noradrenaline and derivatives thereof).

Similarly, the term "β₃ adrenoceptors" will be understood to have its usual meaning in the art which, generally stated, includes receptors known by alternative terminologies including atypical β adrenoceptor, β₃ adrenoceptors receptor, β₃ adrenergic receptor, and β₃ adrenaline receptor.

In the context of this specification, an agonist is any substance, agent or drug that stimulates physiological activity of a cell receptor that is normally capable of being stimulated by a naturally occurring or endogenous regulatory substance. It is to be understood that, in the context of this specification, the term "agonist" includes partial agonists in which the substance, agent or drug may be only partly as effective as an endogenous regulatory substance. It will be understood that, in the context of this specification, the term "agonist" includes any substance, agent or drug that acts directly on a receptor, including those which have affinity for a receptor, and any substance, agent or drug which may act indirectly on a receptor, such as through one or more intermediate substance(s) or pathways, to stimulate activity of the receptor.

In the context of this specification, the term "β₃ agonist" and "β₃ adrenoceptor agonist" includes within its scope any agent capable of activating a β₃ adrenoceptor, in particular any agent capable of activating a human β₃ adrenoceptor.

In the context of this specification, the term "selective", when used in the context of a β adrenoceptor agonist or β adrenoceptor antagonist, means that the agonist or antagonist has preferential, but not necessarily sole, activity toward one or more of the β₁, β₂, or β₃, adrenoceptors. For example, a β₃ adrenoceptor selective agonist has preferential stimulatory activity toward the β₃ adrenoceptor. For example, a β₁ adrenoceptor selective antagonist has preferential inhibitory effect toward the β₁ adrenoceptor.

In the context of this specification, the term "β blocker" means any substance, agent or drug that has antagonist or inhibitory activity toward a β₁ adrenoceptor and/or a β₂ adrenoceptor.

In the context of this specification, "heart failure" (HF) includes both chronic and acute heart failure. Thus, for example, it is expected that the method of the invention is suitable for treatment of chronic heart failure and for treatment of acute heart failure. It will also be understood that, in the context of this specification the term "heart failure" includes symptomatic heart failure and asymptomatic heart failure, and therefore includes susceptibility to acute or chronic heart failure. It will also be understood that, in the context of this specification, the term "heart failure" includes systolic heart failure and diastolic heart failure.

In the context of this specification, the term "patient" includes humans and individuals of any species of social, economic or research importance including but not limited to members of the genus ovine, bovine, equine, porcine, feline, canine, primates, rodents.

In the context of this specification, the term "combined with" and similar terms such as "in conjunction with" when used in relation to a therapeutic regime means that each of the drugs and other therapeutic agent(s), such as agonists and antagonists, is used in the treatment of an individual and that each of the drugs and other therapeutic agents in the "combined" therapeutic regime may be administered to the individual simultaneously with one or more of the other agents in the therapeutic regime, or may be administered to the individual at a different time to one or more of the other agents in the therapeutic regime. That is, the term "combined with" and similar terms such as "in conjunction with" when used in relation to a therapeutic regime may mean that any one or more of the drugs or other agents may be physically combined prior to administration to the patient, and it will be understood that the term also includes administration of the one or more drugs and other therapeutic agents as separate agents not in prior physical combination.

In the context of this specification, the term "comprising" means "including principally, but not necessarily solely". Furthermore, variations of the word "comprising", such as "comprise" and "comprises", have correspondingly varied meanings.

### Abbreviations

In the context of this specification, "HF" is an abbreviation for heart failure.

In the context of this specification, "SR" is an abbreviation for sarcoplasmic reticulum.

In the context of this specification, "CHF" is an abbreviation for congestive heart failure.

In the context of this specification, "LV" is an abbreviation for left ventricular.

In the context of this specification, "Ip" is an abbreviation for the measurable cellular transmembranous current generated by the Na,K-pump in experimental situations using a patch clamp technique.

In the context of this specification, "NMG.Cl" is an abbreviation for N-methyl-D-glucamine.

In the context of this specification, "ESPVR" is an abbreviation for left ventricular end systolic pressure-volume relationship.

In the context of this specification, "PKA" is an abbreviation for cAMP-activated protein kinase.

In the context of this specification, "H-89" is an abbreviation for (N-[2-(p-Bromocinnamylamino)ethyl]-5-isoquinolinesulfonamide dihydrochloride.

### Figure Legends

**Figure 1** demonstrates the effects of the β₃ adrenoceptor selective agonist BRL 37344 on myocardial Na,K-pump-mediated current at agonist concentrations of 1 nM, 10 nM and 100 nM.
**Figure 2** demonstrates the effects of the β₃ adrenoceptor selective agonist BRL 37344 on myocardial Na,K-pump-mediated current at an agonist concentration of 100 nM in the absence and presence of the β₁/β₂ blocker nadolol at 1 µM.
**Figure 3** demonstrates the effects of the β₃ adrenoceptor selective agonist BRL 37344 on myocardial Na,K-pump-mediated current at an agonist concentration of 10 nM in the absence of extracellular Na⁺ (NaCl was replaced by NMG.Cl).
**Figure 4** demonstrates the effects of the naturally occurring catecholamine, noradrenaline (NA), on myocardial Na,K-pump-mediated current at an agonist concentration of 10 nM in the absence and presence of nadolol (1 µM; a β₁/β₂ blocker) and in the absence and presence of H-89 (0.5 µM; an inhibitor of PKA).
**Figure 5** demonstrates the effects of selective activation of β₁/β₂ adrenergic-coupled intracellular messenger pathway, PKA, using the cAMP analogue 6-Bnz-cAMP (100 µM) in the absence and presence of H-89 (0.5 µM).
**Figure 6** demonstrates the effects of BRL 37344 administration in the sheep heart failure model (Huang *et al*., 2004), before and after induction of heart failure, using the left ventricular end systolic pressure-volume relationship (ESPVR) as an index of cardiac performance.

### Detailed Description of the Invention and Best Mode

The invention will now be described in more detail, including, by way of illustration only, with respect to the examples which follow.

This invention is a completely new pharmacological approach to management of patients, especially human patients, with conditions characterised by abnormalities of myocardial cell ion levels, in particular abnormalities of cellular Na⁺, K⁺ or Ca²⁺ ionsThis invention provides a completely new pharmacological approach to the management of patients with symptomatic heart failure (HF).

The invention is based on the inventors' surprising discovery that a myocardial cell surface receptor, the β₃ adrenoceptor, activates the membrane Na⁺-K⁺ pump to extrude Na⁺ from cells. Since cardiac myocyte Na⁺ is implicated in the pathophysiology of heart failure the discovery allows a rational design of a new pharmacological approach to treatment of HF using β₃ adrenoceptors activating drugs (β₃ agonists).

All cells, including cardiac myocytes, are characterised by a difference in electrical potential between the inside of the cell and the outside, separated by the cell membrane. The cell membrane in cardiac myocytes is called the sarcolemma. During the cardiac cycles the membrane potential undergoes cyclical changes with the inside transiently changing from a negative potential in the resting state to a positive potential. This change in membrane potential is called the cardiac action potential. Events during the cardiac action potential are crucial for myocyte contraction and hence pumping function of the heart. The interaction between the cardiac action potential and contraction is often referred to as "excitation-contraction coupling".

The change in membrane potential during the cardiac action potential activates specialised channels in the membrane to transiently become permeable to Ca²⁺ ions. The ions enter the cell from an area of high concentration on the outside to one of low concentration on the inside. The Ca²⁺ that enters serves as a "trigger" to release Ca²⁺ stored in intracellular structures called the sarcoplasmic reticulum (SR). The Ca²⁺ that enters *via* channels or is released from the SR combines to interact with contractile proteins of the cell and initiate contraction of the heart.

Relaxation of the heart is just as important as contraction. Relaxation depends on removal of Ca²⁺. This occurs by an energy-dependent re-uptake of Ca²⁺ into the SR and by its extrusion from the inside of the cell to the surrounding outside milieu, across the cell membrane. Extrusion of Ca²⁺ across the cell membrane occurs *via* an energy-dependent Ca²⁺ pump and via a Na⁺-Ca²⁺ exchange mechanism that transports Ca²⁺ out in exchange for Na⁺ that is transported in. In most mammalian species, including man, the Na⁺-Ca²⁺ exchange is quantitatively the most important pathway for the extrusion of Ca²⁺.

Abnormalities in cellular handling of Ca²⁺ during excitation-contraction coupling are pivotal in the pathogenesis of HF. Typically, there is a delay in the decline in intracellular Ca²⁺ after the transient increase that initiates contraction. Many mechanisms have been proposed, including abnormalities in the function and/or abundance of the membrane proteins that mediate re-uptake of Ca²⁺ into the SR or exchange with Na⁺ across the sarcolemmal membrane.

It has recently been demonstrated that raised intracellular levels of Na⁺ (and hence a decrease in the electrochemical driving force that extrudes Ca²⁺ in exchange for Na⁺) is a pivotal abnormality in HF. This applies to both animals with experimentally induced HF and humans with the clinical condition. The raised intracellular Na⁺ levels may be a major cause of abnormal cellular Ca²⁺ handling because of the role of the transmembrane Na⁺ gradient in cellular extrusion of Ca²⁺ *via* Na⁺-Ca²⁺ exchange.

Thus the high Na⁺ levels may be responsible for abnormalities in contraction and they may activate gene programming present earlier in life and contribute to abnormal cardiac myocyte growth and adverse cardiac remodelling. While the presence of high intracellular Na⁺ levels in HF and myocardial hypertrophy are now widely accepted, the mechanism for the increase is uncertain. Enhanced Na⁺ influx as well as diminished Na⁺ efflux have been proposed.

### The role of myocardial Na⁺ and Na⁺, K⁺-pumps in HF

As described above, the "uphill" transport of Ca²⁺ ions out of cells, against their electrochemical gradient, depends on the energy stored in an oppositely directed electrochemical gradient for Na⁺ (high concentration on the outside, low concentration on the inside). The transmembrane Na⁺ gradient, in turn, is maintained by the Na⁺-K⁺ pump, effectively the only export route for Na⁺. The pump transports 3 Na⁺ ions out of cells in exchange for 2 K⁺ ions that are transported in. It is an energy-dependent process that depends on hydrolysis of ATP for its operation, hence it is often referred to as the Na⁺-K⁺-ATPase.

The role of the Na⁺-K⁺ pump in excitation-contraction coupling has been recognised for approximately 45 years. The best known example of its involvement relates to the effect of cardiac glycosides, *e.g.* digoxin. This group of drugs are highly specific inhibitors of the Na⁺-K⁺ pump. Exposure of normal cardiac tissue to cardiac glycosides causes an increase in intracellular Na⁺, an increase in Ca²⁺ and hence enhanced contractility. This scheme has formed the foundation for use of cardiac glycosides in the treatment of HF. However, only modest glycoside-induced increases in intracellular Na⁺ enhance contraction, and an increase in intracellular Na⁺ of a few mM beyond "normal" levels start having an adverse effect on contraction. As described above intracellular Na⁺ is already raised in HF and further increases are not likely to be beneficial. In agreement with this, although treatment with cardiac glycosides may be beneficial in terms of temporary symptomatic improvement, it does not improve survival in HF and even has an adverse effect on mortality in some subsets of patients.

The present invention is directed to measures with the potential to cause Na⁺-K⁺ pump *stimulation* in HF and other conditions. Prior to the present invention the use of β₃-adrenoceptor selective agonists had not been explored as a potential Na⁺-K⁺ pump activator.

### β₃ adrenoceptors

β₃ adrenoceptors are expressed in many tissues, including the heart, and are present in many species including humans. Prior to the present invention the primary clinical and pharmacological interest in β₃ adrenoceptors has been related to a potential role of β₃ adrenoceptors agonists ("activators") in the treatment of obesity and type II diabetes mellitus, based on findings in rodents that β₃ adrenoceptors agonists induce an increase in metabolic rate, a lipolytic effect and an increased insulin sensitivity.

Studies on isolated cardiac tissue, including tissue from humans, have established that β₃ adrenoceptor selective agonists mediates a negative inotropic effect, *i.e.* a decrease in contractility. This is in marked contrast to β₁ and β₂ receptors agonists that mediate positive inotropy, *i.e*. an increase in contractility. The mechanism for the negative inotropic effect of β₃-adrenoceptors has not been firmly established, but has been suggested to be mediated by calcium channel blockage.

β₃ adrenoceptors are upregulated with the development of HF. It has been suggested that β₃ adrenoceptors have adverse effects on the progression of HF. Gauthier *et al.* (1998) thus suggested that catecholamine-activated β₃ adrenoceptor-mediated pathways may lead to myocardial dysfunction. It has also been proposed that the negative inotropic effect mediated by β₃ adrenoceptors is maladaptive and aggravates systolic dysfunction, particularly with severe HF (Moniotte *et al.,* 2001). Cheng *et al.* (2001) suggest that the β₃ adrenoceptor contributes to progression of cardiac dysfunction in HF. Morimoto *et al* (2004) reported that blocking β₃ adrenoceptor in a model of cardiomyocyte dysfunction in heart failure improved left ventricular (LV) contraction and relaxation and that stimulation of β₃ adrenoceptor by the high levels of catecholamines in HF contributes to the impaired LV contraction and relaxation. Morimoto *et al* propose that the clinical implications of their results include that β₃ adrenoceptor blockade may provide a pharmacological method of inotropic support for the failing heart and that chronic β₃ adrenoceptor blockade might have favourable effects in HF.

In view of the role of the Na⁺-K⁺ pump in cardiac contraction (pump inhibition expected to have positive inotropic effect, pump stimulation expected to have a negative inotropic effect), the present inventors tested the hypothesis that the β₃ adrenoceptor is linked to activation of the Na⁺-K⁺ pump. The inventors describe herein that, surprisingly, the β₃ adrenoceptor can mediate an increase in the activity of sarcolemmal Na⁺-K⁺ pump in isolated cardiac myocytes. This is the first demonstration of an effect of β₃ adrenoceptors on regulation of myocardial Na⁺-K⁺ pump activity.

This β₃ adrenoceptor-mediated stimulation of the Na,K-pump will increase the cellular efflux of Na⁺. Since raised levels of cardiac myocyte Na⁺ is a hallmark of heart failure and myocardial hypertrophy such stimulation of Na⁺ efflux may be beneficial. In support of this proposal, the well documented effective treatments with ACE-inhibitors and aldosterone antagonists are associated with Na-K pump stimulation in cardiac myocytes (Hool, LC et al., Am J Physiol 271:C172-180, 1996, Mihailidou AS et al., Circ Res 86: 37-42, 2000). The effects of these groups of drugs have the property in common of counteracting inhibitory effects of the naturally occurring hormones angiotensin II and aldosterone on the Na-K pump. This invention describes a new receptor-mediated method of directly stimulating the pump rather than merely preventing inhibition. The effects of the method may be additive to that of the already existing treatments. Initially a decrease in intracellular Na⁺ levels may cause some deterioration in myocardial contractility. However, any possible initial deterioration should be followed by improvement in parallel with a gradual reduction in intracellular Na⁺ concentration. Thus, the discovery of stimulation of the myocardial Na,K-pump by beta 3 adrenoceptor selective agonists provides a completely new pharmacological approach to the management of patients with heart failure or myocardial hypertrophy using beta 3 adrenoceptor activating drugs (beta 3 adrenoceptor selective agonists).

Accordingly, described herein is the use of one or more β₃ adrenoceptor selective agonists in the treatment of HF or myocardial hypertrophy.

### β₃ adrenoceptor selective agonists

β₃ adrenoceptor selective agonists are known in the art and include, but are not limited to, the β₃ adrenoceptor agonist groups arylethanolamines, aryloxypropanolamines, trimetoquinols including, but not limited to, BRL37344, , N-5984, (R)-N-[4-[2-[[2-Hydroxy-2-(pyridin-3-yl)ethyl]amino]ethyl]phenyl]- 4-[4-(4-trifluoromethylphenyl)thiazol-2-yl]benzenesulfonamide (L-796568), (R)-N-[4-[2-[[2-hydroxy-2-(3-pyridinyl)- ethyl]amino]ethyl]phenyl]-1-(4-octylthiazol-2-yl)-5-indolinesulfonamide (L-755507), L-770,644, L-766,892, L-757,793, LY-377604, , SR 58611A, , are also contemplated in the invention.

It will be appreciated that the method of the invention contemplates the use of any suitable β₃ adrenoceptor selective agonist and that specific groups and compounds are stated herein by way of exemplification and not by way of limitation.

Any salt (acid or base salt) and hydrates, polymorphs, etc, forms of those agents can also be used.

The β₃ adrenoceptor agonist may be selective, including, but not limited to, BRL37344, and selective compounds disclosed in US Patent No. 6,686,372 to Crowell et al entitled "Selective β₃ adrenergic agonists".

Also known in the art are compounds having β₃ adrenoceptor agonist activity and β₁ and/or β₂ adrenoceptor antagonist activity. Described herein is the use of such compounds in the treatment of HF or myocardial hypertrophy.

The use of β-adrenoceptor, blockade in HF (through the use of antagonists of β₁ and/or β₂ adrenoceptor) is already firmly established as beneficial in the treatment of HF. Described herein is the use of a β₃ adrenoceptor selective agonist in the treatment of HF or myocardial hypertrophy. It is also expected that a combined therapeutic regime, comprising use of a β₃ adrenoceptor selective agonist in conjunction with one or more β₁-and or β₂-adrenoceptor antagonists may provide improved therapeutic outcomes.

Accordingly, provided herein is a method for the treatment of HF or myocardial hypertrophy wherein one or more β₃ adrenoceptor selective agonists is administered to an individual in conjunction with one or more β₁- and or β₂-adrenoceptor antagonists. β-adrenoceptor antagonists in this aspect of the invention may be those with an established role in the treatment of HF.

### β₁- and or β₂-adrenoceptor antagonists

Compounds and agents having β₁- and or β₂-adrenoceptor antagonist activity are known in the art and include, but are not limited to, alprenolol, amosulalol, atenolol, betaxolol, bethanidine, bevantolol, bisoprolol, bopindolol, bufuralol, bunitrolol, bupranolol, butafilolol, carazolol, carteolol, carvedilol, celiprolol, cetamolol, dilevalol, epanolol, indenolol, labetalol, mepindolol, metipranolol, metoprolol, moprolol, nadolol, nebivolol, nipradilol, oxprenolol, penbutolol, pindolol, pronethalol, propranolol, sotalol, sulfinalol, talinolol, tertatolol, tilisolol, timolol, toliprolol, and the like. β₃-adrenoceptor antagonists are also described in US Patent No. 4,678,786 to Roe et al entitled "Pharmaceutical compositions having β-adrenoceptor antagonist activity employing pyridazinone derivatives". β-adrenoceptor antagonists are described more fully in the literature, such as in Goodman and Gilman, The Pharmacological Basis of Therapeutics (9th Edition), McGraw-Hill, 1995; and in The Merck Index, (13th Edition), Merck & Co., Whitehouse Station, N.J., USA, 2004.

The β₁- and or β₂-adrenoceptor antagonist may be any relatively non-selective P-adrenoceptor antagonist including, but not limited to, alprenolol, oxprenolol, sotalol and timolol. The β₁- and or β₂-adrenoceptor antagonist may be a selective β₁-adrenoceptor antagonist, including, but not limited to, nebivolol, atenolol, bisopronol, betaxolol, metoprolol, practolol and CGP20712A. The β-adrenoceptor antagonist may be a selective β₂-adrenoceptor antagonist, including, but not limited to, ICI118551.

The β₁- and or β₂-adrenoceptor antagonist, at therapeutic amounts may have relatively low, or no, affinity for β₃-adrenoceptor and so leave the β₃-adrenoceptor relatively unblocked. Such an antagonist includes, but is not limited to, metoprolol.

It will be appreciated that the method of the invention contemplates the use of any suitable agent having β₁- and or β₂-adrenoceptor antagonist activity and that specific groups and compounds are stated herein by way of exemplification and not by way of limitation.

It will be appreciated that reference to the agents includes all salt (acid or base salt) and hydrates, polymorphs, etc, forms of those agents.

### Centrally acting sympatolytic agents

Also known in the art is the use of centrally acting sympatolytic agents in the treatment of HF. It is expected that combined use of β₃ adrenoceptor selective agonists with a centrally acting sympatolytic agent may also provide improved therapeutic outcome for the treatment of HF.

Accordingly, described herein is a method for the treatment of HF or myocardial hypertrophy wherein one or more β₃ adrenoceptor selective agonists is administered to an individual in combination therapy with one or more centrally acting sympatolytic agents. Centrally-acting sympatolytic agents are known in the art and include, but are not limited to, those listed in The Merck Index, (13th Edition), Merck & Co., Whitehouse Station, N.J., USA, 2004.

### Treatment regime

The most appropriate treatment regime for any particular patient may be determined by the treating physician and will depend upon a variety of factors including: the disorder being treated and the severity of the disorder; activity of the compound or agent employed; the composition employed; the age, body weight, general health, sex and diet of the patient; the time of administration; the route of administration; the rate of sequestration of the agent or compound; the duration of the treatment; drugs used in combination or coincidental with the treatment, together with other related factors well known in medicine.

Based on theoretical expectations, the present inventors envisage that a clinically significant negative inotropic effect may be experienced if β₃ adrenoceptor selective agonists were to be given to patients with HF. As such, a patient's condition may transiently deteriorate before their condition improves, as is the case with commencement of treatment with β-adrenoceptor blockade and ACE inhibitors in HF.

In the animal heart failure model exemplified herein (see Example 6) no such initial deterioration was observed.

Accordingly, in the method and, where desirable to reduce actual or anticipated transient deterioration of the condition of the patient, treatment with one or more β₃ adrenoceptor selective agonists may be used in a patient already stabilised on conventional treatment such as with ACE-inhibitors, including but not limited to captopril, enalapril and lisonopril, aldosterone antagonists and β₁- and or β₂-adrenoceptor antagonists. Furthermore, the clinical practice of "starting low and going slow" of antagonist treatment may also be followed for β₃ adrenoceptor selective agonist treatment.

The start low go slow principle means to start at such a low dose that adverse effects are very unlikely, but - on the other hand - no significant beneficial effects are expected either. After a period, which may be days or weeks, when no or only minor adverse effects have been observed a minor up-titration of the dose is performed. This scheme is continued until adverse effects are seen, for example ortostatic hypotension during beta-blocker up-titration, or until a recommended target dose is reached. It may take weeks to months from commencement until target dose is reached.

ACE-inhibitor, beta-blocker and aldosterone antagonist, such as spironolactone and eplerenone, are three classes of drugs proven to reduce symptoms and improve survival in patients with HF. As a further example of a treatment regime, current pharmacological management of HF includes the "add-on" principle. In such a regime, the patient may start treatment with an ACE inhibitor which may be titrated up to a certain level. Then treatment with a beta-blocker may be commenced (added-on) and titrated up to a certain level and then treatment with spironolactone is commenced. Conditions related to treatment incorporating the "add-on" principle are also included in a treatment regime using one or more β₃ adrenoceptor selective agonists.

Thus, the administration of one or more β₃ adrenoceptor selective agonists may be as an "add-on", in which a patient may be treated with a drug from each of the three classes ACE-inhibitor, beta-blocker and aldosterone antagonist, and before, during or after that treatment, treatment with one or more β₃ adrenoceptor selective agonists may be commenced, preferably using the start low go slow principle. Consequently, it will be appreciated that in this context the term "add-on" refers to an additional therapeutic integer (the β₃ adrenoceptor selective agonist); it does not mean that the β₃ adrenoceptor selective agonist must be added as the last drug. The order and composition of the specific drugs and drug classes in the combination therapy may be determined by the skilled addressee, and may include, for example where the therapeutic regime involves the administration of four drug classes, the β₃ adrenoceptor selective agonist may be the first, second, third, or fourth drug to be administered.

As a further example of a treatment regime, the condition of a patient may be at least partially stabilized prior to administration of the one or more β₃ adrenoceptor selective agonists. Furthermore, the condition of a patient may be at least partially stabilized prior to commencement of a method of the invention. Either of such treatment regimes may be referred to as first stabilizing a patient.

First stabilising a patient includes, for example, where treatment with ACE-inhibitors and beta-blockers is not commenced in HF when the patient's cardiac function is severely compromised, typically with a low blood pressure and a low cardiac output. A reason for postponing commencement of the therapy is that these drugs may initially deteriorate the condition by inducing further reductions in blood pressure or cardiac output. On this basis the patient's condition is first stabilised, for example by optimisation of intravascular fluid volume (euvolemia), over a period of, for example, a few days before the "initial minor deterioration" is expected to be tolerated.

Conditions related to commencement of treatment with β₃ adrenoceptor selective agonists after first stabilizing a patient may be similar to the situation for ACE-inhibitor and beta-blocker treatment.

As a further example of a treatment regime, a β₃ adrenoceptor selective agonist may be the first drug to be administered in the treatment of heart failure without any prior medication or stabilisation. For example, commencement of β₃ adrenoceptor selective agonist treatment in a patient with only, or mainly, diastolic cardiac dysfunction or in patients with myocardial hypertrophy without significantly reduced cardiac systolic function is not expected to induce any initial side effects.

The compounds and agents proposed for the present invention may be administered as compositions therapeutically. In a therapeutic application, compositions are administered, for example, to a patient already having symptomatic heart failure, in an amount sufficient to effectively treat the patient
Preventive applications are also described wherein compositions are administered to, for example, a patient having myocardial hypertrophy in an amount sufficient to effectively treat the patient thereby at least slowing or preventing disease progression. The composition should thus provide a quantity of the compound or agent sufficient to effectively treat the patient.

The therapeutically effective dose level for any particular patient will depend upon a variety of factors including: the disorder being treated and the severity of the disorder; activity of the compound or agent employed; the composition employed; the age, body weight, general health, sex and diet of the patient; the time of administration; the route of administration; the rate of sequestration of the agent or compound; the duration of the treatment; drugs used in combination or coincidental with the treatment, together with other related factors well known in medicine.

One skilled in the art would be able, by routine experimentation, to determine an effective, non-toxic amount of the β₃ adrenoceptor selective agonist, and other agents where appropriate, which would be required to treat the condition.

Generally, an effective dosage is expected to be in the range of about 0.00001mg to about 1000mg per kg body weight per 24 hours; typically in the range of about 0.0001mg to about 1000mg per kg body weight per 24 hours; typically, about 0.001mg to about 750mg per kg body weight per 24 hours; about 0.01mg to about 500mg per kg body weight per 24 hours; about 0.1mg to about 500mg per kg body weight per 24 hours; about 0.1mg to about 250mg per kg body weight per 24 hours; about 1.0mg to about 250mg per kg body weight per 24 hours. More typically, an effective dose range is expected to be in the range about 1.0mg to about 200mg per kg body weight per 24 hours; about 1.0mg to about 100mg per kg body weight per 24 hours; about 1.0mg to about 50mg per kg body weight per 24 hours; about 1.0mg to about 25mg per kg body weight per 24 hours; about 5.0mg to about 50mg per kg body weight per 24 hours; about 5.0mg to about 20mg per kg body weight per 24 hours; about 5.0mg to about 15mg per kg body weight per 24 hours.

Alternatively, an effective dosage may be up to about 500mg/m². Generally, an effective dosage is expected to be in the range of about 25 to about 500mg/m², preferably about 25 to about 350mg/m², more preferably about 25 to about 300mg/m², still more preferably about 25 to about 250mg/m², even more preferably about 50 to about 250mg/m², and still even more preferably about 75 to about 150mg/m².

Typically, in therapeutic applications, the treatment would be for the duration of the disease state.

Further, it will be apparent to one of ordinary skill in the art that the optimal quantity and spacing of individual dosages and, where combination therapy is used, optimal quantity and spacing of administration of the various agents of the combination therapy, will be determined by the nature and extent of the disease state being treated, the form, route and site of administration, and the nature of the particular individual being treated. Also, such optimum conditions can be determined by conventional techniques.

It will also be apparent to one of ordinary skill in the art that the optimal course of treatment, such as, the number of doses of the composition or compositions given per day for a defined number of days, can be ascertained by those skilled in the art using conventional course of treatment determination tests.

### Pharmaceutical compositions

In general, suitable compositions may be prepared according to methods which are known to those of ordinary skill in the art and accordingly may include a pharmaceutically acceptable carrier, diluent, excipient and/or adjuvant.

These compositions can be administered by standard routes. In general, the compositions may be administered by the parenteral (e.g., intravenous, intraspinal, subcutaneous or intramuscular), oral or topical route. Preferably administration is by the parenteral or oral route. More preferably administration is by the oral route.

The carriers, diluents, excipients and adjuvants must be "acceptable" in terms of being compatible with the other ingredients of the composition, and not deleterious to the recipient thereof.

Examples of pharmaceutically acceptable carriers or diluents are demineralised or distilled water; saline solution; vegetable based oils such as peanut oil, safflower oil, olive oil, cottonseed oil, maize oil, sesame oils, arachis oil or coconut oil; silicone oils, including polysiloxanes, such as methyl polysiloxane, phenyl polysiloxane and methylphenyl polysolpoxane; volatile silicones; mineral oils such as liquid paraffin, soft paraffin or squalane; cellulose derivatives such as methyl cellulose, ethyl cellulose, carboxymethylcellulose, sodium carboxymethylcellulose or hydroxypropylmethylcellulose; lower alkanols, for example ethanol or iso-propanol; lower aralkanols; lower polyalkylene glycols or lower alkylene glycols, for example polyethylene glycol, polypropylene glycol, ethylene glycol, propylene glycol, 1,3-butylene glycol or glycerin; fatty acid esters such as isopropyl palmitate, isopropyl myristate or ethyl oleate; polyvinylpyrridone; agar; carrageenan; gum tragacanth or gum acacia, and petroleum jelly. Typically, the carrier or carriers will form from 10% to 99.9% by weight of the compositions.

The composition may include agents which increase the bioavailability or therapeutic duration of the active compound or compounds.

The compositions of the invention may be in a form suitable for administration by injection, in the form of a formulation suitable for oral ingestion (such as capsules, tablets, caplets, elixirs, for example), in the form of an ointment, cream or lotion suitable for topical administration, in a form suitable for delivery as an eye drop, in an aerosol form suitable for administration by inhalation, such as by intranasal inhalation or oral inhalation, in a form suitable for parenteral administration, that is, subcutaneous, intramuscular or intravenous injection.

For administration as an injectable solution or suspension, non-toxic parenterally acceptable diluents or carriers can include, Ringer's solution, isotonic saline, phosphate buffered saline, ethanol and 1,2 propylene glycol.

Some examples of suitable carriers, diluents, excipients and adjuvants for oral use include peanut oil, liquid paraffin, sodium carboxymethylcellulose, methylcellulose, sodium alginate, gum acacia, gum tragacanth, dextrose, sucrose, sorbitol, mannitol, gelatine and lecithin. In addition these oral formulations may contain suitable flavouring and colourings agents. When used in capsule form the capsules may be coated with compounds such as glyceryl monostearate or glyceryl distearate which delay disintegration.

Adjuvants typically include emollients, emulsifiers, thickening agents, preservatives, bactericides and buffering agents.

Solid forms for oral administration may contain binders acceptable in human and veterinary pharmaceutical practice, sweeteners, disintegrating agents, diluents, flavourings, coating agents, preservatives, lubricants and/or time delay agents. Suitable binders include gum acacia, gelatine, corn starch, gum tragacanth, sodium alginate, carboxymethylcellulose or polyethylene glycol. Suitable sweeteners include sucrose, lactose, glucose, aspartame or saccharine. Suitable disintegrating agents include corn starch, methylcellulose, polyvinylpyrrolidone, guar gum, xanthan gum, bentonite, alginic acid or agar. Suitable diluents include lactose, sorbitol, mannitol, dextrose, kaolin, cellulose, calcium carbonate, calcium silicate or dicalcium phosphate. Suitable flavouring agents include peppermint oil, oil of wintergreen, cherry, orange or raspberry flavouring. Suitable coating agents include polymers or copolymers of acrylic acid and/or methacrylic acid and/or their esters, waxes, fatty alcohols, zein, shellac or gluten. Suitable preservatives include sodium benzoate, vitamin E, alpha-tocopherol, ascorbic acid, methyl paraben, propyl paraben or sodium bisulphite. Suitable lubricants include magnesium stearate, stearic acid, sodium oleate, sodium chloride or talc. Suitable time delay agents include glyceryl monostearate or glyceryl distearate.

Liquid forms for oral administration may contain, in addition to the above agents, a liquid carrier. Suitable liquid carriers include water, oils such as olive oil, peanut oil, sesame oil, sunflower oil, safflower oil, arachis oil, coconut oil, liquid paraffin, ethylene glycol, propylene glycol, polyethylene glycol, ethanol, propanol, isopropanol, glycerol, fatty alcohols, triglycerides or mixtures thereof.

Suspensions for oral administration may further comprise dispersing agents and/or suspending agents. Suitable suspending agents include sodium carboxymethylcellulose, methylcellulose, hydroxypropylmethyl-cellulose, poly-vinyl-pyrrolidone, sodium alginate or acetyl alcohol. Suitable dispersing agents include lecithin, polyoxyethylene esters of fatty acids such as stearic acid, polyoxyethylene sorbitol mono- or di-oleate, -stearate or - laurate, polyoxyethylene sorbitan mono- or di-oleate, -stearate or -laurate and the like.

The emulsions for oral administration may further comprise one or more emulsifying agents. Suitable emulsifying agents include dispersing agents as exemplified above or natural gums such as guar gum, gum acacia or gum tragacanth.

Methods for preparing parenterally administrable compositions are apparent to those skilled in the art, and are described in more detail in, for example, Remington's Pharmaceutical Science, 15th ed., Mack Publishing Company, Easton, Pa., hereby incorporated by reference herein.

The topical formulations of the present invention, comprise an active ingredient together with one or more acceptable carriers, and optionally any other therapeutic ingredients. Formulations suitable for topical administration include liquid or semi-liquid preparations suitable for penetration through the skin to the site of where treatment is required, such as liniments, lotions, creams, ointments or pastes, and drops suitable for administration to the eye, ear or nose. Formulations suitable for topical administration may be provided as a transdermal patch.

Drops according to the present invention may comprise sterile aqueous or oily solutions or suspensions. These may be prepared by dissolving the active ingredient in an aqueous solution of a bactericidal and/or fungicidal agent and/or any other suitable preservative, and optionally including a surface active agent. The resulting solution may then be clarified by filtration, transferred to a suitable container and sterilised. Sterilisation may be achieved by: autoclaving or maintaining at 90°C-100°C for half an hour, or by filtration, followed by transfer to a container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Lotions according to the present invention include those suitable for application to the skin or eye. An eye lotion may comprise a sterile aqueous solution optionally containing a bactericide and may be prepared by methods similar to those described above in relation to the preparation of drops. Lotions or liniments for application to the skin may also include an agent to hasten drying and to cool the skin, such as an alcohol or acetone, and/or a moisturiser such as glycerol, or oil such as castor oil or arachis oil.

Creams, ointments or pastes according to the present invention are semi-solid formulations of the active ingredient for external application. They may be made by mixing the active ingredient in finely-divided or powdered form, alone or in solution or suspension in an aqueous or non-aqueous fluid, with a greasy or non-greasy basis. The basis may comprise hydrocarbons such as hard, soft or liquid paraffin, glycerol, beeswax, a metallic soap; a mucilage; an oil of natural origin such as almond, corn, arachis, castor or olive oil; wool fat or its derivatives, or a fatty acid such as stearic or oleic acid together with an alcohol such as propylene glycol or macrogols.

The composition may incorporate any suitable surfactant such as an anionic, cationic or non-ionic surfactant such as sorbitan esters or polyoxyethylene derivatives thereof. Suspending agents such as natural gums, cellulose derivatives or inorganic materials such as silicaceous silicas, and other ingredients such as lanolin, may also be included.

The compositions may also be administered in the form of liposomes. Liposomes are generally derived from phospholipids or other lipid substances, and are formed by mono- or multi-lamellar hydrated liquid crystals that are dispersed in an aqueous medium. Any non-toxic, physiologically acceptable and metabolisable lipid capable of forming liposomes can be used. The compositions in liposome form may contain stabilisers, preservatives, excipients and the like. The preferred lipids are the phospholipids and the phosphatidyl cholines (lecithins), both natural and synthetic. Methods to form liposomes are known in the art, and in relation to this specific reference is made to: Prescott, Ed., Methods in Cell Biology, Volume XIV, Academic Press, New York, N.Y. (1976), p. 33 et seq., the contents of which is incorporated herein by reference.

### Examples

The examples are intended to serve to illustrate this invention and should not be construed as limiting the general nature of the disclosure of the description throughout this specification.

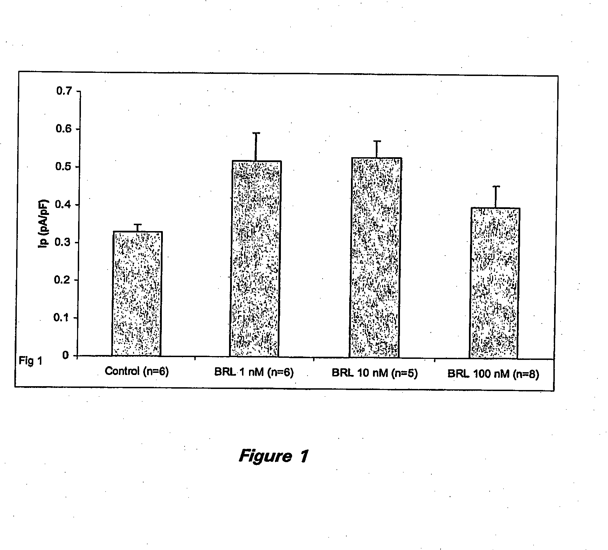

Figures 1, 2 and 3 below summarise experiments demonstrating how the β₃ adrenoceptor-selective agonist BRL 37344 stimulates the membrane Na⁺-K⁺ pump in isolated rabbit cardiac myocytes. The experiments suggest that pump stimulation is reduced when high concentrations of the drug is used and that this reversal of a pump stimulatory effect is likely to be due to activation of β₁ and/or β₂ adrenoceptors at high BRL 37344 concentrations. The experiments demonstrate that the BRL 37344-induced increase in pump activity is due to direct Na⁺-K⁺ pump stimulation rather than secondary to a BRL 37344-induced increase in transmembrane Na⁺ influx and stimulation of the pump by a resulting increase in the intracellular Na⁺ concentration.

### Example 1

The whole-cell patch clamp technique (Hamill *et al,* 1981; Buhagier *et al,* 2004; Hansen *et al,* 2002) was used to examine the effect of β₃ adrenoceptor stimulation on the Na⁺-K⁺ pump in isolated cardiac myocytes.

With the whole-cell patch clamp technique myocytes are suspended in a tissue bath mounted on the stage of an inverted microscope. A fine glass pipette is attached to the cell membrane and gentle suction applied to rupture the membrane patch directly under the tip of the glass pipette. This allows the intracellular compartment of the myocyte to be perfused with solution contained in the patch pipette. It also allows access of drugs and compounds to the intracellular compartment. Control of the composition of the extracellular solution in the tissue bath is achievable. The technique also allows control of membrane voltage and measurement of total membrane current.

The Na⁺-K⁺ pump's 3Na⁺:2K⁺ exchange ratio generates a current (Iₚ) that can be measured as the shift in holding current of the patch-clamped myocyte that is induced by pump blockade with 100 µM ouabain.

In the whole-cell configuration the bath was perfused with modified Tyrode's solution which contained (in mmol/L) NaCl 140; KCl 5.6; CaCl₂ 2.16; MgCl₂ 1; NaH₂PO₄ 0.44; glucose 10 and Na-glutamate 9; N-2-hydroxyethyl piperazine-N'-2-ethene-sulphonic acid (HEPES) 10. The solution was titrated to a pH of 7.40 ± 0.01 at 35°C with NaOH.

For measurement of Ip the system was switched to a superfusate that usually was identical to the modified Tyrode's solution except that it was nominally Ca²⁺-free and contained 0.2 mmol/L CdCl₂ and 2 mmol/L BaCl₂.

Wide-tipped patch pipettes (4-5 µm) were filled with solutions containing (in mmol/L) HEPES 5; MgATP 2; ethylene glycol-bis(β-aminoethyl ether)-N,N,N',N'-tetraacetic acid (EGTA) 5; KCl 70 mmol/L. Osmotic balance was maintained with 80 mmol/L tetramethylammonium chloride (TMA-Cl). The solutions were titrated to a pH of 7.05 ± 0.01 at 35°C with 1 mmol/L TMA-OH. Ip was identified at a holding potential of -40 mV as the shift in holding current induced by Na⁺-K⁺ pump blockade with 100 µmol/L ouabain 10-15 minutes after the whole-cell configuration had been established. Ip is reported normalized for membrane capacitance and hence cell size. Details of patch pipette characteristics and equipment and criteria for defining Ip have been reported previously (Buhagiar *et al,* 1999).

The effects of different concentrations of BRL 37344 (Sigma Aldrich) on Na⁺-K⁺ pump current (Iₚ) was determined. The Na concentration in the pipette solution was 10 mM, *i.e.* at a near-physiological level. Figure 1 shows that in comparison with normal controls, BRL 37344 significantly stimulates the Na,K-pump at all three concentration levels. At the highest level (100 nM BRL 37344) an apparent lower activity was observed in comparison with activity observed with 10 nM. This is likely to be due to unspecific binding of BRL 37344 to β₁ and/or β₂ adrenoceptors at higher concentrations. β₁ and β₂ adrenoceptors agonists are expected to inhibit the myocardial Na,K-pump. Mean, SEM and number of experiments (n) values are indicated.

### Example 2

See the description of the whole-cell patch clamp technique described in Example 1.

This experiment examined if the apparent decrease in stimulation of the Na,K-pump with exposure to 100 nM BRL 37344 compared to the stimulation seen with 10 nM BRL 37344 might be caused by stimulation of β₁ and/or β₂ adrenoceptors by the higher BRL 37344 concentration.

The effect of 100 nM BRL 37344 in the superfusate in the absence and in the presence of the combined β₁ and β₂ adrenoceptor blocker nadolol was measured in myocytes.

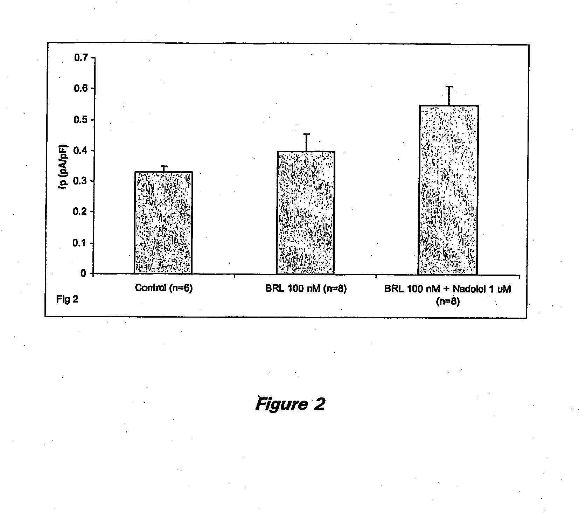

Figure 2 shows that blockage of β₁ and β₂ adrenoceptors with nadolol in the presence of 100 nM BRL 37344 increased, although not quite significantly, the activity of the Na,K-pump as compared to the stimulation seen with 100 nM BRL 37344 alone.

This suggests that the attenuated Na,K-pump stimulation with the high BRL 37344 concentration (see Figure 1) was a result of BRL 37344 stimulation of the β₁ and/or β₂ adrenoceptors. These results also confirm that the pump stimulation observed in response to the β₃ adrenoceptor selective agonist at the lower concentrations of 1 nM and 10 nM was mediated by β₃ rather than β₁ and/or β₂ adrenoceptors and suggest that β₁ and/or β₂ adrenoceptor activation may reduce Na,K-pump activity. Mean, SEM and number of experiments (n) values are indicated.

### Example 3

The purpose of this experiment was to investigate if the β₃ adrenoceptor selective agonist BRL 37344-induced stimulation of the Na,K-pump is caused by, or is secondary to, increased influx of Na⁺ into the cell. Increased intracellular Na⁺ stimulates the Na⁺-K⁺ pump. If the effect of β₃ adrenoceptor were secondary to an increase of Na⁺ influx rather than primarily Na⁺-K⁺ pump stimulation, then β₃ adrenoceptor selective agonists would be expected to be harmful in heart disease.

The whole-cell patch clamp technique was similar to that described above except that extracellular Na⁺ was eliminated (NaCl was replaced with N-methyl-D-glucamine (NMG.Cl)) to rule out any possible influx of Na⁺ across the cell membrane.

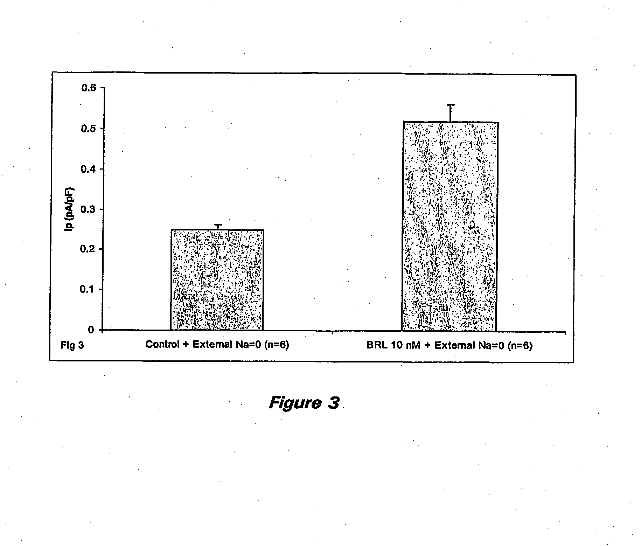

Figure 3 shows that BRL 37344-induced stimulation of the Na,K-pump persisted in the absence of extracellular Na⁺. This result is consistent with stimulation having been due to an intrinsic change in Na⁺-K⁺ pump function rather than due to BRL 37344-induced transsarcolemmal Na⁺ influx and so is consistent with direct Na⁺-K⁺ pump stimulation rather than a secondary effect.

### Example 4

In the Examples 1, 2, and 3 the effect of the synthetic selective β₃ adrenergic agonist BRL 37344 on the sarcolemmal Na⁺-K⁺ pump was examined. In this example the effect of the naturally occurring catecholamine, noradrenaline (NA) was examined. The whole-cell patch clamp technique was used as described in Example 1.

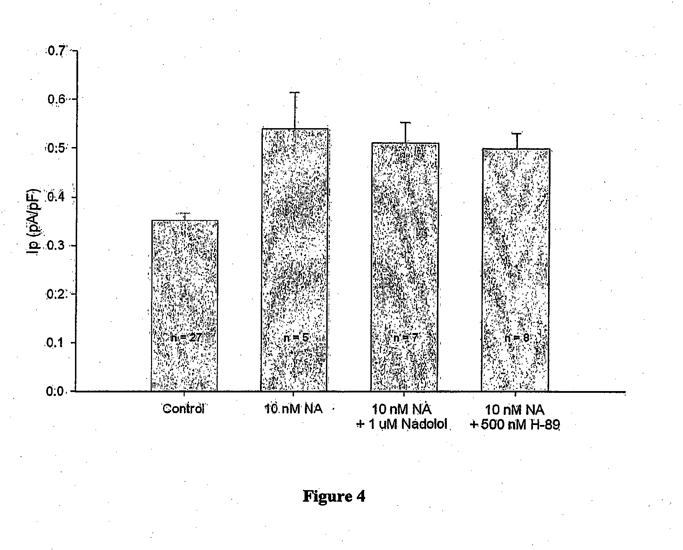

Isolated cardiac myocytes were exposed to 10 nM NA and Ip was measured. As shown in Figure 4, NA induced an increase in Iₚ. We next examined the effect of NA on Iₚ when β₁- and β₂ adrenergic receptors were blocked with nadolol. Myocytes were exposed to 10 nM NA and to 1 µM nadolol. Figure 4 demonstrates that the NA-induced increase in Ip persisted in the presence of nadolol. This strongly suggests that the increase is not mediated by β₁/β₂ adrenergic receptors.

To obtain further support for this conclusion the effect of blocking the intracellular messenger pathway classically activated by β₁/β₂ adrenergic receptors was examined. Activation of the β₁/β₂ adrenergic receptors results in intracellular synthesis of cAMP and subsequent activation cAMP-activated protein kinase (*a.k.a.* PKA). Myocytes were patch clamped using a patch pipette solution that contained 0.5 µM H-89 (N-[2-(p-Bromocinnamylamino)ethyl]-5-isoquinolinesulfonamide dihydrochloride (Calbiochem), an inhibitor of PKA. H-89 at this concentration abolishes PKA-mediated Na⁺-K⁺ pump inhibition (William *et al*., 2003). As shown in Figure 4, the NA-induced increase in Ip persisted with PKA blockade.

### Example 5

In a further set of experiments the effect of selectively activating β₁/β₂ adrenergic receptor-coupled intracellular messenger pathways was investigated. Since it is very difficult to experimentally selectively activate β₁/β₂ adrenergic membrane receptors with extracellular compounds we chose to directly activate the intracellular pathway known to be activated by the receptors, ie the cAMP-activated protein kinase (PKA). The cAMP analog 6-Bnz-cAMP activates PKA.

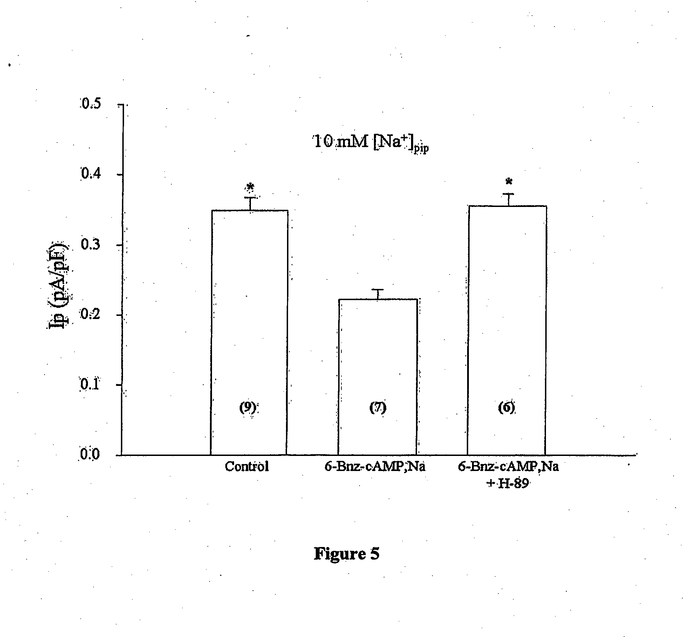

100 µM of 6-Bnz-cAMP was included in the patch pipette solutions that perfused the intracellular compartment. The myocytes were exposed to NA-free control superfusate. As shown in Figure 5, the analogue 6-Bnz-cAMP included in the pipette solution induced a significant decrease in Iₚ. To ascertain that the 6-Bnz-cAMP-induced decrease in Iₚ was mediated by PKA we also measured Iₚ using pipette solutions that included 6-Bnz-cAMP as well as H-89. The latter was expected to inhibit 6-Bnz-cAMP-activated PKA. Results are included in Figure 5 and demonstrate that H-89 (0.5 µM) reversed the 6-Bnz-cAMP-induced decrease in Iₚ.

Taken together, the results presented here indicate that activation of β₁/β₂ adrenergic receptors cause Na⁺-K⁺ pump inhibition while activation of β₃ adrenergic receptors causes Na⁺-K⁺ pump stimulation. This provides an explanation for the already established efficacy of β₁/β₂ adrenergic receptor blockers in human heart failure, and it identifies the β₃ adrenergic receptor as an entirely new drug target. Stimulation of the receptor is expected to promote Na⁺ export from Na⁺-overloaded cells in heart failure, thereby providing a beneficial therapeutic effect.

The role of the β₃ adrenergic receptor is supported by the inability of β₁/β₂ blockade with nadolol to block pump stimulation, by the persistence of stimulation when intracellular messenger pathways classically activated by β₁/β₂ receptors are blocked and by the effect of the selective β₃ adrenergic-selective agonist BRL 37344 to induce stimulation of Iₚ.

### Example 6

### Effect of BRL 37344 on cardiac performance in sheep heart failure model

To examine the effect of acute activation of the β₃-receptor we used a model of ischaemic cardiomyopathy with severe heart failure induced by repeated coronary microembolisation in sheep (Huang, Y et al., Am J Physiol 286: H2141-50, 2004). The model is stable and reflects the human condition well (Huang *et al,* 2004).

BRL 37344 was administered by intravenous infusion over a period of 8 min, starting with the lowest dose of the protocol. After an additional 5 min pressure-volume relationships were determined as described (Huang *et al.,* 2004). The procedure was then repeated in an identical fashion using a larger dose of BRL 37344. The left ventricular end systolic pressure-volume relationship (ESPVR) was used as an index of cardiac performance. ESPVR is relatively independent of confounding factors such as pre- and after-load and heart rate, and is widely accepted as a good index of left ventricular function.

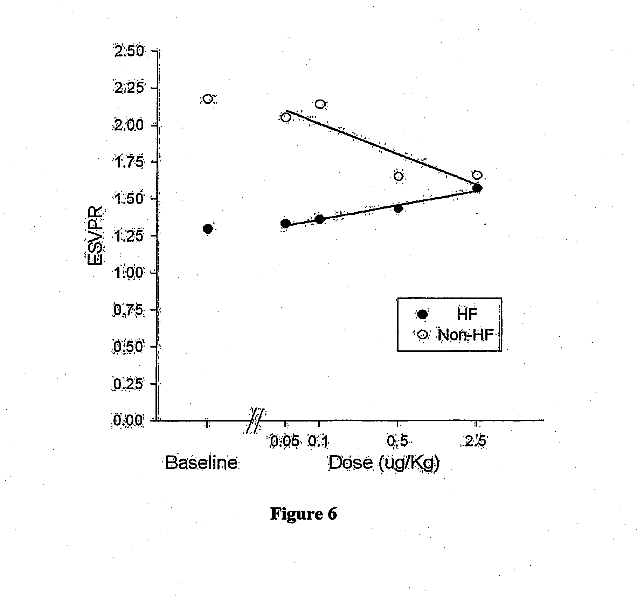

Results are shown in Figure 6, both before and after induction of heart failure. The independent variable on the abscissa indicates the dose in microgram BRL 37344 per kg weight of the sheep. Note the logarithmic increment in dose. The mean of ESPVR measured in 3 different sheep before and after induction of severe heart failure is shown on the ordinate. A linear regression analysis of a logarithmic transformation of the dose of BRL 37344 vs. ESPVR indicated a trend towards a negative slope in normal sheep consistent with a dose-dependent deterioration in left ventricular function. In contrast, the slope was significantly positive after heart failure had been induced in the sheep indicating a dose-dependent improvement in left ventricular function with administration of BRL 37344. It is concluded that β₃ adrenergic receptors activation has acute beneficial effects in heart failure, which is consistent with the expectations of the inventors based on the results presented herein for *in vitro* stimulation of the Na⁺-K⁺ pump.

### Summary

A change in the intracellular Na⁺ concentration generally has opposite effects under normal physiological conditions and under conditions of heart failure. In the normal heart, an increase in the intracellular Na⁺ concentration causes an increase in the intracellular Ca²⁺ concentration and hence an enhancement of contractility. However, only modest increases in intracellular Na⁺ improve cardiac performance, and an increase of a few mM beyond "normal" has adverse effects. In heart failure, intracellular Na⁺ is already raised, and further increases impair performance. The present inventors describe, for the first time herein, a method for directly activating the cell membrane pump that extrudes Na⁺ from the cells. As supported by the examples herein, this is expected to reduce Na⁺ towards normal in the Na⁺ overloaded cells and hence enhance cardiac performance. The method to activate Na⁺ extrusion from cells involves the use of one or more β₃ adrenoceptor selective agonists.

Any description of prior art documents herein, or statements herein derived from or based on those documents, is not an admission that the documents or derived statements are part of the common general knowledge of the relevant art in Australia or elsewhere.

While the invention has been described in the manner and detail as above, it will be appreciated by persons skilled in the art that numerous variations and/or modifications including various omissions, substitutions, and/or changes in form or detail may be made to the invention as shown in the specific embodiments without departing from the spirit or scope of the invention as broadly described. The present embodiments are, therefore, to be considered in all respects as illustrative and not restrictive.

### List of References

Bloom, J. and Claus, T. Drugs Future 1994, 19:23.
Buhagiar KA, Hansen PS, Gray DF, Mihailidou AS and Rasmussen HH. Angiotensin regulates the selectivity of the Na+-K+ pump for intracellular Na+. Am J Physiol 277: C461-C468, 1999.
Buhagiar KA, Hansen PS, Kong BY, Clarke RJ, Fernandes C and Rasmussen HH. Dietary cholesterol alters Na+/K+ selectivity at intracellular Na+/K+ pump sites in cardiac myocytes. Am J Physiol Cell Physiol 286: C398-C405, 2004.
Bundgaard H, Havndrup O, Andersen PS, Larsen LA, Brandt NJ, Vuust J, Kjeldsen K, Christiansen M. Familial hypertrophic cardiomyopathy associated with a novel missense mutation affecting the ATP-binding region of the cardiac beta-myosin heavy chain. J Mol Cell Cardiol. 1999, 31(4):745-50.
Cantello, B. and Smith, S., Drugs Future 1991, 16:797.
Cecchi, R., et al., Eur. J. Med. Chem. 1994, 29:259.
Cheng HJ, Zhang ZS, Onishi K, Ukai T, Sane DC and Cheng CP. Upregulation of functional beta(3)-adrenergic receptor in the failing canine myocardium. Circ Res 89: 599-606, 2001.
Gauthier C, Leblais V, Kobzik L, Trochu JN, Khandoudi N, Bril A, Balligand JL and Le Marec H. The negative inotropic effect of beta3-adrenoceptor stimulation is mediated by activation of a nitric oxide synthase pathway in human ventricle. J Clin Invest 102: 1377-1384, 1998.
Gheorghiade, M. et al 2003, Braunwauld, Heart Diseases, 6th Edition.
Goodman and Gilman, The Pharmacological Basis of Therapeutics (9th Edition), McGraw-Hill, 1995.
Hamill OP, Marty A, Neher E, Sakmann B and Sigworth FJ. Improved patch-clamp techniques for high-resolution current recording from cells and cell-free membrane patches. Pflugers Arch 391: 85-100, 1981.
Hansen PS, Buhagiar KA, Kong BY, Clarke RJ, Gray DF and Rasmussen HH. Dependence of Na+-K+ pump current-voltage relationship on intracellular Na+, K+, and Cs+ in rabbit cardiac myocytes. Am JPhysiol Cell Physiol 283: C1511-C1521, 2002.
Harada, H et al, Novel and potent human and rat β3-Adrenergic receptor agonists containing substituted 3-indolylalkylamines, Bioorganic & Medicinal Chemistry Letters 13 (2003): 1301-1305..
Hool, LC et al., Am J Physiol 271:C172-180, 1996.
Huang, Y., et al., Remodeling of the chronic severely failing ischemic sheep heart after coronary microembolization: functional, energetic, structural, and cellular responses. Am JPhysiol Heart Circ Physiol 286: H2141-50, 2004.
Jong P, Vowinckel E, Liu PP, Gong Y and Tu JV. Prognosis and determinants of survival in patients newly hospitalized for heart failure: a population-based study. Arch Intern Med 162: 1689-1694, 2002.
Levy D, Kenchaiah S, Larson MG, Benjamin EJ, Kupka MJ, Ho KK, Murabito JM and Vasan RS. Long-term trends in the incidence of and survival with heart failure. N Engl J Med 347: 1397-1402, 2002.
Mihailidou AS et al., Circ Res 86: 37-42, 2000.
Moniotte S, Kobzik L, Feron O, Trochu JN, Gauthier C and Balligand JL. Upregulation of beta(3)-adrenoceptors and altered contractile response to inotropic amines in human failing myocardium. Circulation 103: 1649-1655, 2001.
Morimoto A, Hasegawa H, Cheng HJ, Little WC and Cheng CP. Endogenous {beta}3-Adrenoceptor Activation Contributes to Left Ventricular and Cardiomyocyte Dysfunction in Heart Failure. Am J Physiol Heart Circ Physiol .: 2004.
Prescott, Ed., Methods in Cell Biology, Volume XIV, Academic Press, New York, N.Y.(1976).
Remington's Pharmaceutical Science, 15th ed., Mack Publishing Company, Easton, Pa.
Souza, J., et al., Curr. Pharm. Des. 2001, 7:1433.
The Merck Index, (13th Edition), Merck & Co., Whitehouse Station, N.J., USA, 2004.
Vakili BA, Okin PM, Devereux RB. Prognostic implications of left ventricular hypertrophy. Am Heart J. 2001 Mar;141(3):334-41.
Verdecchia P, Angeli F, Borgioni C, Gattobigio R, de Simone G, Devereux RB, Porcellati, C. Changes in cardiovascular risk by reduction of left ventricular mass in hypertension: a meta-analysis. 2003, Am JHypertens. 2003 Nov;16(11 Pt 1):895-9.
Weber, A. Annu. Rep. Med. Chem. 1998, 33:193.
Weyer, C., et al., Diabetes Metab. 1999, 25:11.
William, M., Vein, J., and Rasmussen, H. Biphasic effect of atrial natriuretic peptide on the sarcolemmal sodium pump. Biophys.J. Suppl 84-2, 191a. 2003.
Yanagisawa T, Sato T, Yamada H, Sukegawa J, Nunoki K. Selectivity and potency of agonists for the three subtypes of cloned human beta-adrenoceptors expressed in Chinese hamster ovary cells. Tohoku J Exp Med. 2000 Nov;192(3):181-93.

## Claims

1. A composition comprising a therapeutically effective amount of one or more β₃ adrenoceptor selective agonists having preferential stimulatory activity toward the β₃ andrenoreceptor for use in the treatment of a human suffering from symptomatic heart failure, the use further comprising use of one or more β₃ blockers.

2. The composition for use according to claim 1 wherein the human has one or more clinical symptoms of heart failure.

3. The composition for use according to claim 1 wherein the β₃ adrenoceptor selective agonist is selected from the group consisting of arylethanolamines, aryloxypropanolamines and trimetoquinols.

4. The composition for use according to claim 1 wherein the β₃ adrenoceptor selective agonist is BRL37344.

5. The composition for use according to any preceding claim wherein the P blocker is nadolol.

6. The composition for use according to any preceding claim wherein the β blocker is a β₁ and/or β₂ adrenoceptor antagonist.

7. The composition for use according to any preceding claim wherein the use of one or more β blockers is prior to, simultaneously with or subsequent to the use of the one or more β₃ adrenoceptor selective agonists.

8. The composition for use according to any preceding claim further comprising at least partially stabilizing the human prior to the use of said β₃ adrenoceptor selective agonist.

9. The composition for use according to claim 8 wherein said stabilizing comprises treatment with one or more compounds selected from the group consisting of ACE-inhibitors, aldosterone antagonists and β adrenoceptor antagonists.

## Patentansprüche

1. Zusammensetzung, umfassend eine therapeutisch wirksame Menge eines oder mehrerer Agonisten mit einer Selektivität gegenüber dem ß₃-Adrenozeptor, die bevorzugt eine stimulatorische Wirkung auf den β₃-Adrenorezeptor ausüben, zur Verwendung bei der Behandlung eines menschlichen Patienten, der an symptomatischer Herzinsuffizienz leidet, wobei die Verwendung des Weiteren die Verwendung eines oder mehrerer β-Blocker umfasst.

2. Zusammensetzung zur Verwendung nach Anspruch 1, wobei sich bei dem menschlichen Patienten ein oder mehrere klinische Symptome der Herzinsuffizienz zeigen.

3. Zusammensetzung zur Verwendung nach Anspruch 1, wobei der Agonist mit einer Selektivität gegenüber dem β₃-Adrenozeptor ausgewählt ist aus der Gruppe bestehend aus Arylethanolaminen, Aryloxypropanolaminen und Trimetochinolen.

4. Zusammensetzung zur Verwendung nach Anspruch 1, wobei es sich bei dem Agonisten mit einer Selektivität gegenüber dem β₃-Adrenozeptor um BRL37344 handelt.

5. Zusammensetzung zur Verwendung nach einem der vorhergehenden Ansprüche, wobei es sich bei dem β-Blocker um Nadolol handelt.

6. Zusammensetzung zur Verwendung nach einem der vorhergehenden Ansprüche, wobei es sich bei dem β-Blocker um einen Antagonisten des β₁- und/oder β₂-Adrenozeptors handelt.

7. Zusammensetzung zur Verwendung nach einem der vorhergehenden Ansprüche, wobei die Verwendung des einen oder der mehreren β-Blocker entweder vor, gleichzeitig mit oder nach der Verwendung des einen oder der mehreren Agonisten mit einer Selektivität gegenüber dem β₃-Adrenozeptor erfolgt.

8. Zusammensetzung zur Verwendung nach einem der vorhergehenden Ansprüche, des Weiteren umfassend eine zumindest teilweise Stabilisierung des menschlichen Patienten vor der Verwendung des Agonisten mit einer Selektivität gegenüber dem β₃-Adrenozeptor.

9. Zusammensetzung zur Verwendung nach Anspruch 8, wobei die Stabilisierung eine Behandlung mit einer oder mehreren Verbindungen umfasst, die ausgewählt sind aus der Gruppe bestehend aus ACE-Inhibitoren, Aldosteron-Antagonisten und β-Adrenozeptor-Antagonisten.

## Revendications

1. Composition comprenant une quantité thérapeutiquement efficace d'un ou de plusieurs agonistes sélectifs de récepteur β₃-adrénergique ayant une activité stimulante préférentielle envers le β₃-adrénorécepteur, destinée à une utilisation dans le traitement d'un être humain souffrant d'une défaillance cardiaque symptomatique, l'utilisation comprenant en outre l'utilisation d'un ou de plusieurs β-bloquants.

2. Composition destinée à une utilisation selon la revendication 1, où l'être humain présente un ou plusieurs symptômes cliniques de défaillance cardiaque.

3. Composition destinée à une utilisation selon la revendication 1, où l'agoniste sélectif de récepteur β₃-adrénergique est sélectionné parmi le groupe constitué d'aryléthanolamines, d'aryloxypropanolamines et de trimétoquinols.

4. Composition destinée à une utilisation selon la revendication 1, où l'agoniste sélectif de récepteur β₃-adrénergique est BRL37344.

5. Composition destinée à une utilisation selon l'une quelconque des revendications précédentes, où le β-bloquant est le nadolol.

6. Composition destinée à une utilisation selon l'une quelconque des revendications précédentes, où le β-bloquant est un antagoniste de récepteur β₁-adrénergique et/ou β₂-adrénergique.

7. Composition destinée à une utilisation selon l'une quelconque des revendications précédentes, où l'utilisation d'un ou de plusieurs β-bloquants a lieu avant, de manière simultanée avec ou après l'utilisation des un ou plusieurs agonistes sélectifs de récepteur β₃-adrénergique.

8. Composition destinée à une utilisation selon l'une quelconque des revendications précédentes, comprenant en outre une stabilisation au moins de manière partielle de l'être humain avant l'utilisation dudit agoniste sélectif de récepteur β₃-adrénergique.

9. Composition destinée à une utilisation selon la revendication 8, où ladite stabilisation comprend un traitement avec un ou plusieurs composés sélectionnés parmi le groupe constitué d'inhibiteurs d'enzyme de conversion de l'angiotensine, d'antagonistes d'aldostérone et d'antagonistes de récepteur β-adrénergique.
